# EUROPEAN PATENT APPLICATION

(11) **EP 1 845 159 A1**
(43) Date of publication of application: **17.10.2007**
(21) Application number: 06712984.1
(22) Date of filing: 03.02.2006
(51) Int. Cl.: C12N 15/09, A01K 67/027, C07K 14/47, C12Q 1/68

(54) **METHOD OF DETERMINING GENE RELATING TO FAVORABLE BEEF TASTE AND TEXTURE**

(30) Priority: 04.02.2005 JP 2005028384
(71) Applicant: The New Industry Research Organization, Kobe-shi, Hyogo 650-0047 (JP)
(72) Inventor: TSUZI, Soichi National Univ. Corp. Kobe Univ., Kobe-shi Hyogo 6578501 (JP); MANNEN, Hideyuki National Univ. Corp. Kobe Univ., Kobe-shi Hyogo 6578501 (JP)
(74) Representative: Behnisch, Werner
(86) International application number: PCT/JP2006/301841
(87) International publication number: WO 2006/082916

(57) **Abstract**

We paid attention to SREBP-1 gene, and investigated the relation between this gene and the unsaturated fatty acid content in beef fat. As a result, we found a genotype of this gene; i.e., "L type" having a longer fifth intron and "S type" whose fifth intron was shorter by 84 bases than the above "L type", and that the unsaturated fatty acid content was associated with the above genotype. A method for evaluating the unsaturated fatty acid content in beef fat, on the basis of the above SREBP-1 genotype, is applicable to evaluation of beef quality such as taste and texture and therefore, industrially useful.

## Description

### Technical field

The present invention relates to a method for evaluating quality of beef such as taste and texture through genotyping, and more specifically relates to a method for evaluating, on the basis of the genotype of sterol regulatory element binding protein (SREBP-1), whether or not it is a cattle, from which better quality of beef with better taste and texture can be produced. The present invention is useful for various fields such as livestock industry (feeding, breeding, reproduction, etc.), beef production, processing and distribution.

### Background art

It is known that the content of unsaturated fatty acid in beef fat is associated with quality of beef, such as the beef taste and texture. Generally, if the content of unsaturated fatty acid is high with low melting point, the beef is considered to have good quality, which gives good taste and texture.

However, in order to judge whether the beef has such quality, there was no other way but to depend on the subjective (sensuous) method, in which the beef was actually eaten and evaluated. In other words, there was no conventional method, which was more objective, simple and efficient, like the judgment approach of predicting quality of beef, simply based on the genotype of a specific bovine gene.

By the way, stearoyl-CoA desaturase (SCD) is known as an enzyme which desaturates beef fat. This enzyme desaturates stearoyl-CoA which plays an important role for in vivo biosynthesis of lipids and their degradation. Amino acid sequence and cDNA sequence of bovine stearoyl-CoA (derived from Bos taurus) are shown in DDBJ/EMBL/GenBank databases; Accession number "AB075020".

The inventors disclose, in JP 2004-261014, a method for evaluating the amount of the unsaturated fatty acid content in beef fat, on the basis of a genotype of SCD, to evaluate whether or not it is a cattle, from which better quality of beef with better taste and texture can be produced.

As mentioned above, the content of unsaturated fatty acid in beef fat (in other words, the amount of the unsaturated fatty acid content) is associated with quality of beef, such as the beef taste and texture. If the amount of the unsaturated fatty acid content can be predicted on the basis of the genotype of a specific bovine gene, then it provides a new method which enables simple examination as to quality of beef, such as the beef taste and texture. Such method based on the genotype is useful not only for evaluation and selection of cattle (beef cattle) with good quality of beef, but also for breeding and reproduction of cattle.

Furthermore, the content of unsaturated fatty acid in beef fat is associated with the cholesterol accumulation caused by beef intake, and it is considered one of important features of the cattle, especially in the countries where beef is eaten very often, such as the Europe and the United States of America. Therefore, the above-mentioned method for evaluating the unsaturated fatty acid content based on the genotype, is also useful from the view point of health care.

The above-mentioned judgment method based on the SCD genotype, proposed by the inventors, is useful. However, the development of a new method for evaluating through a different genotype has been expected, because, in some cases, it was difficult to evaluate quality of beef only by the SCD genotype. Such new method can be expected to provide a more excellent evaluation method, by combining with the judgment method based on the SCD genotype.

### Disclosure of the invention

The object of the present invention is (1) to provide a new method for simply and efficiently evaluating the amount of the unsaturated fatty acid content in beef fat on the basis of the genotype of a bovine gene, and (2) to provide a new method for predicting, based on its evaluation, quality of beef, such as the beef taste and texture, more objectively.

The unsaturated fatty acid content is different among the cattle kinds. For example, the unsaturated fatty acid content of Japanese Black Cattle is higher than that of the Holstein-Friesian, and the melting point in beef fat of Japanese Black Cattle is lower. Therefore, Japanese Black Cattle is considered to have good quality, which gives good taste and texture. As a result of analysis, the inventors deduced that the SCD gene expression level may be associated with the difference of the unsaturated fatty acid content. Further analysis revealed that (1) there is a SRE (sterol regulatory element) sequence at the upstream regulatory region of SCD gene, to which SREBP-1 (sterol regulatory element binding protein) binds as a transcription factor, (2) the SREBP-1 gene expression level is associated with the SCD gene expression level, (3) there is a polymorphism in this SREBP-1 gene; i.e., one type (L type) having a longer fifth intron while another type (S type) having a fifth intron shorter by 84 bases than the above L type, and (4) some Japanese Black Cattle have the S type, and the unsaturated fatty acid content of cattle with this S type is higher and its melting point in beef fat is lower. We also clarified the gene sequence and the amino acid sequence of bovine SREBP-1, along with the above analysis. These findings led us to the present invention.

The present invention includes the following industrially useful inventions A) to L).
A) A method for evaluating the amount of the unsaturated fatty acid content in beef fat, on the basis of a genotype of sterol regulatory element binding protein (SREBP-1), to evaluate whether or not it is a cattle, from which better quality of beef with better taste and texture can be produced.
B) The method set forth in A), comprising a step (a) of determining the genotype of SREBP-1 by examination of a polymorphism in a fifth intron of this gene, and a step (b) of evaluating a cattle with a "S type" allele to produce better quality of beef (better taste and texture), compared with a cattle with a "L type" allele, said "S type" allele has a shorter fifth intron while said "L type" allele has a longer fifth intron.
C) The method set forth in B), comprising a step of amplifying a gene region including a deleted portion of 84 bases, related to the polymorphism in the fifth intron, by using a genomic DNA prepared from a test cattle as a template, and a step of determining the genotype of SREBP-1 by a length of an amplified fragment.
D) The method set forth in C), adopting a PCR (polymerase chain reaction) by use of a forward primer having a base sequence shown in SEQ NO.1 and a reverse primer having a base sequence shown in SEQ NO.2 in the amplification step.
E) The method set forth in C), using a genomic DNA prepared from a hair of a test cattle as a template.
F) The method set forth in B), examining the polymorphism in the fifth intron by use of a gene polymorphism detector device such as a DNA chip.
G) A method for evaluating the amount of the unsaturated fatty acid content in beef fat, on the basis of a combination of a genotype of sterol regulatory element binding protein (SREBP-1) and a genotype of stearoyl-CoA desaturase (SCD), to evaluate whether or not it is a cattle, from which better quality of beef with better taste and texture can be produced
H) The method set forth in G), comprising a step of evaluating a cattle to produce better quality of beef (better taste and texture), if the cattle has three or more alleles, as a total number of a number of "S type" alleles among the SREBP-1 genotype and a number of "A-type" alleles among the SCD genotype.
I) A bovine-derived SREBP-1 protein indicated by (a) or (b) below:
   (a) a protein having an amino acid sequence of SEQ NO.6;
   (b) a protein having an amino acid sequence obtained by deletion, replacement, and/or addition of one or several amino acids in the amino acid sequence of SEQ NO.6 and having a transcriptional regulatory activity by binding a SRE sequence.
J) A bovine-derived SREBP-1 gene, encoding any one protein set forth in I).
K) The gene set forth in J), said gene being a DNA indicated by (a) or (b) below:
   (a) a DNA having a base sequence ranging from the 22nd base to the 3324th base, among a base sequence of SEQ NO.5;
   (b) a DNA that has a base sequence hybridized under stringent conditions to a DNA having a base sequence that is complementary to at least 100 base sequence of SEQ NO.5, and that encodes a protein having a transcriptional regulatory activity by binding a SRE sequence.
L) A transgenic cattle, produced by introducing the gene set forth in K).

### Effect of the invention

According to the present invention, it is possible to evaluate not only the unsaturated fatty acid content, but also quality of beef such as taste and texture, by examining whether the SREBP-1 genotype is L type or S type. One example of easy examination is a PCR method using a sample prepared from a cattle hair, in order to determine the cattle has L type or S type. More excellent judgment is available by combining the judgment method of the present invention with the judgment method based on the SCD genotype.

The unsaturated fatty acid content in beef fat is associated with not only quality of beef, but also the cholesterol accumulation caused by beef intake, and it is considered one of important features of the cattle, especially in the countries where beef is eaten very often, such as the Europe and the United States of America. The present invention is useful for various fields such as identification of cattle, selection of stud bull, livestock industry (feeding, breeding, reproduction, etc.), beef production, processing and distribution.

### Brief description of the drawings

FIG.1 is a graph showing the relation between the SCD gene expression level and the content of mono-unsaturated fatty acid (MUFA) in the intermuscular adipose tissue.
FIG.2 is a graph showing the result of examining a time change in the SCD gene expression level of each cattle kind.
FIG.3 is a graph showing the result of examining the relation between the MUFA content and the SCD gene expression level, about plural samples of each cattle kind, which were collected at different time.
FIG.4 is a figure showing a transcriptional regulatory sequence in the upstream regulatory region of SCD gene.
FIG.5 is a graph showing the relation between the SCD gene expression level and the SREBP-1 gene expression level.
FIG.6 is a figure explaining a genomic structure of SREBP-1 gene and a deleted portion of the fifth intron.
FIG.7 is a figure showing the result of judging the SREBP-1 genotype, about each cattle kind of Japanese Black Cattle and the Holstein-Friesian.
FIG.8 is a figure showing the result of judging the SREBP-1 genotype, and measuring the unsaturated fatty acid content and the melting point of each genotype.

### Sequence Listing Free Text

SEQ NO.1: Forward primer sequence for typing bovine SREBP-1 gene.
SEQ NO.2: Reverse primer sequence for typing bovine SREBP-1 gene.
SEQ NO.3: Sequence of a deleted portion in the fifth intron of bovine SREBP-1 gene.
SEQ NO.4: Sequence ranging over the fifth exon, the fifth intron and the sixth exon of bovine SREBP-1 gene.
SEQ NO.5: cDNA sequence and amino acid sequence of bovine SREBP-1.
SEQ NO.6: Amino acid sequence of bovine SREBP-1.
SEQ NO.7: Forward primer sequence for real-time PCR of bovine SCD mRNA.
SEQ NO.8: Reverse primer sequence for real-time PCR of bovine SCD mRNA.
SEQ NO.9: TaqMan™ probe sequence for real-time PCR of bovine SCD mRNA.
SEQ NO.10: Forward primer sequence for real-time PCR of bovine SREBP-1 mRNA.
SEQ NO.11: Reverse primer sequence for real-time PCR of bovine SREBP-1 mRNA.
SEQ NO.12: TaqMan™ probe sequence for real-time PCR of bovine SREBP-1 mRNA.

### Best mode for carrying out the invention

Specific embodiments of the present invention are described in detail below with reference to the drawings.

### (1) The judgment method of the present invention

The present invention is a method for evaluating the amount of the unsaturated fatty acid content in beef fat, based on the genotype of SREBP-1, to evaluate whether or not it is a cattle, from which better quality of beef with better taste and texture can be produced.

Here, "cattle" means the animal belonging to Genus Bos, especially livestock cattle (Bos primigenius). The Europeane cattle (Bos taurus) and the Indian cattle (Bos indicus) are included in this "livestock cattle", and the following breeds of cattle are included in this "Europeane cattle (Bos taurus)"; i.e., Japanese cattle (such as Japanese Black, Japanese Brown, Japanese Polled and Japanese Shorthorn) and Europeane cattle (such as the Holstein-Friesian, the Jersey, the Shorthorn for meat, the Hereford and the Aberdeen Angus). The "cattle" used as a test animal (source of specimen) may be any one of the above beef cattle.

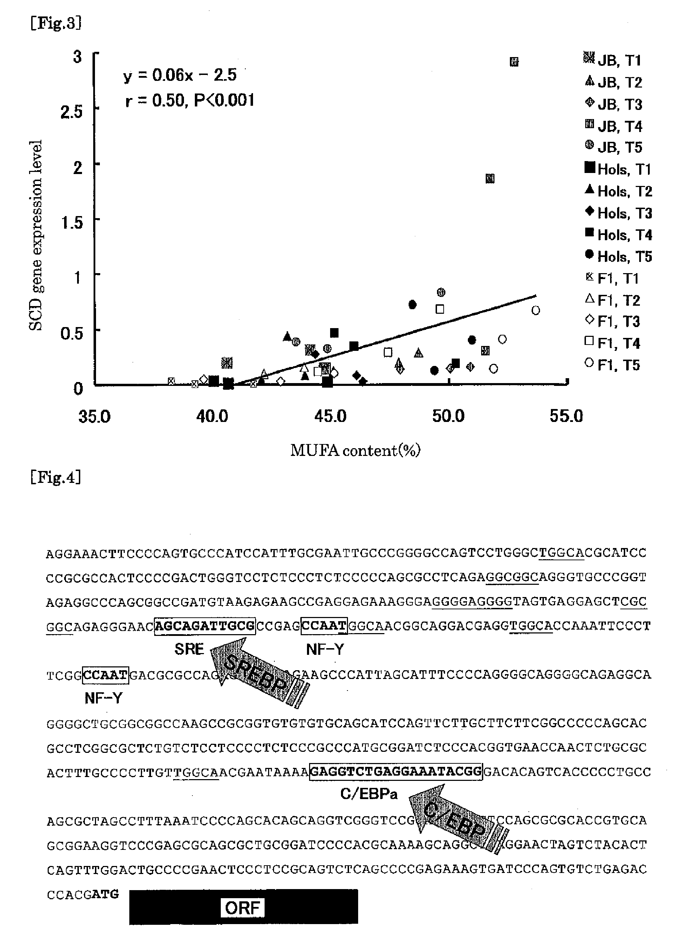

"SREBP-1 (sterol regulatory element binding protein)" is a transcription factor derived from cattle, which binds a SRE sequence (see FIG.4) at the upstream of SCD gene and has a transcriptional regulatory activity. The cDNA sequence of SREBP-1 gene is shown in SEQ NO.5, which was isolated from Japanese Black and decided by the inventors, together with the amino acid sequence encoded by the open reading frame (ORF) of this gene. The amino acid sequence of SREBP-1 protein is also shown in SEQ NO.6.

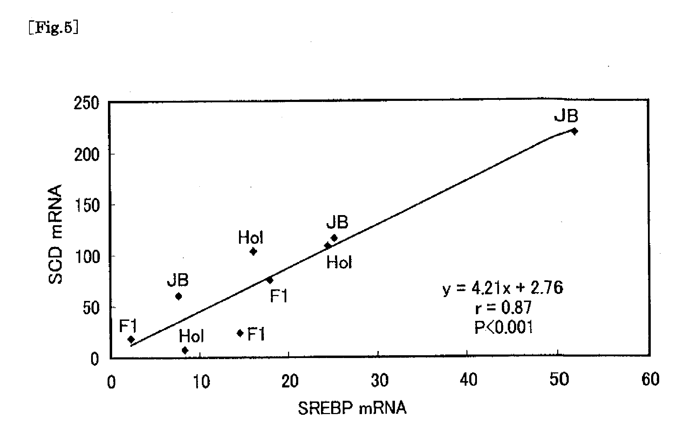

FIG.6 schematically shows a genomic structure of the above SREBP-1 gene. As shown, the SREBP-1 gene comprises 19 exons and 18 introns. In a base sequence of the fifth intron (intron 5), we have found a polymorphism; i.e., one genotype having the underlined 84 bases of FIG.6 while another genotype losing these 84 bases. Hereafter, the underlined 84 bases are conveniently called a "deleted portion". Also, one genotype having a longer fifth intron because of having this deleted portion, is called a "L type" while another genotype having a shorter fifth intron because of losing this deleted portion, is called a "S type". The deleted portion is ranging from the 85th base to the 168th base, in the base sequence of the fifth intron of the "L type".

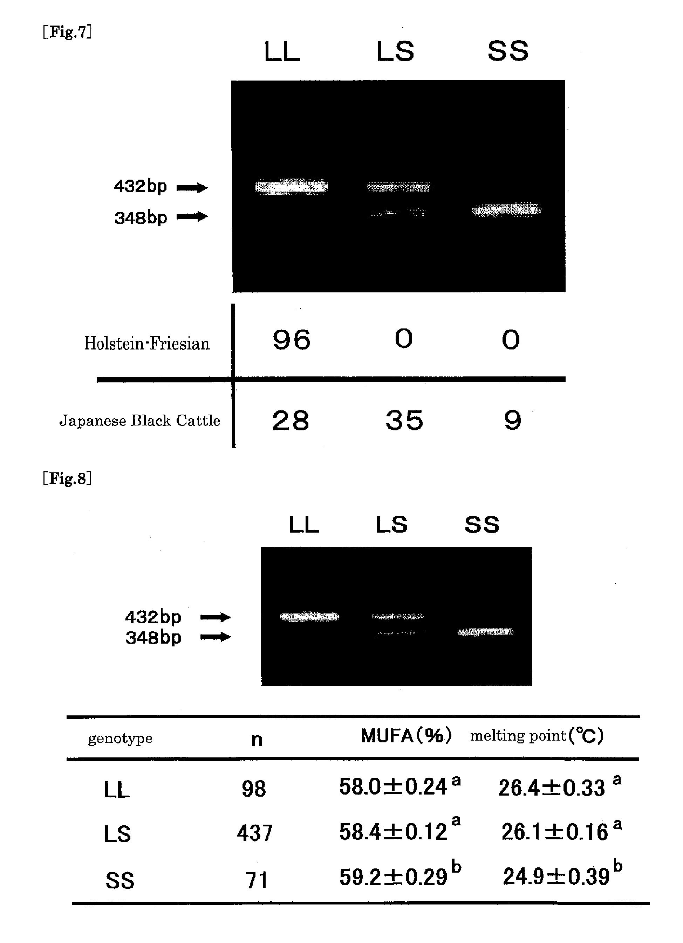

Thus, it was found that the genotype of bovine SREBP-1 gene can be roughly classified into two types of "L type" and "S type". Further investigation revealed that the genotype of bovine SREBP-1 gene is associated with fatty acid composition (in other words, "the content of unsaturated fatty acid in beef fat" or "the amount of the unsaturated fatty acid content"). That is, it was found that there is a significant difference between the above "L type" and "S type" in the content of unsaturated fatty acid in beef fat. Although details of the investigation will be explained in the Example as described later, the percentage of mono-unsaturated fatty acid (MUFA) content in beef fat was the highest when the genotype of bovine SREBP-1 gene was (S/S) having homozygous S type. When the genotype of bovine SREBP-1 gene was (L/S) having heterozygous L and S type, the percentage was higher than that in the case of the genotype (L/L) having homozygous L type (see FIG.8).

As mentioned above, the content of unsaturated fatty acid in beef fat is associated with quality of beef such as taste (flavor) and texture. In general, if the content of unsaturated fatty acid is high, then the melting point of beef fat will be low (FIG.8), and the beef is considered to have good quality, which gives good taste and texture. Therefore, if the genotype of bovine SREBP-1 gene is (S/S), such cattle with (S/S) can be evaluated (estimated) to produce better quality of beef (better taste and texture), compared with the cattle with (L/L). Thus, by examining the genotype of bovine SREBP-1 gene, it becomes possible not only to judge the content of unsaturated fatty acid in beef fat, but also to judge whether it is the cattle, from which high quality of beef (with better taste and texture) can be produced.

In the method of the present invention, the method for examining the genotype of bovine SREBP-1 gene should be not limited, and various known methods can be applied to the method for directly, or indirectly, examining the presence or absence of the above-mentioned "deleted portion". The PCR (Polymerase Chain Reaction) method is an easy and simple method with good accuracy, to examine the presence or absence of the "deleted portion", and to decide the genotype of bovine SREBP-1 gene. The following is a brief explanation of the judgment using this PCR method.

### (2) Method for judging the SREBP-1 genotype by the PCR method

Genetic sample (specimen) for the judgment may be a genomic DNA. The genomic DNA can be extracted and purified by a known method from any organ, tissue or cell of the test cattle (origin of specimen). Extraction of the organ, tissue or cell from the test cattle may be carried out before slaughter, or after slaughter. The cell from the test cattle may be blood (the cell in blood), the cell in the amniotic fluid, or the cultured cell (obtained from extracted tissue etc.). The genomic DNA was prepared from the blood in the Example as described later. The extraction and purification of the genomic DNA may be simplified or omitted, if a target gene region can be amplified by the PCR method without such step.

The genomic DNA prepared by the above method may be used as a template in the PCR method, for the purpose of determination of the genotype (i.e., genotyping) by examining the presence or absence of the above deleted portion. After amplifying a target region including the deleted portion by such PCR method, the genotype of bovine SREBP-1 gene can be judged (determined) according to a length of the amplified fragment.

The above PCR method should be not limited about each condition, used reagents and primers, etc. The following is an explanation about one example of the PCR method, which was adopted in the Example as described later.

The composition of the PCR solution was as follows; genomic DNA 20ng, TaKaRa Ex Taq^{™} polymerase HS 0.5 Unit, 10 x Taq polymerase buffer 2.0µl, 25mM dNTP mix 1.6µl, each 1.0µl of forward primer (10pmol) and reverse primer (10pmol), and ultrapure water added to be total 20µl of solution.

Designed sequences of forward primer and reverse primer are shown in the following (a) and (b) respectively.
(a) 5'- CCACAACGCCATCGAGAAACGCTAC -3' (SEQ No.1)
(b) 5'- GGCCTTCCCTGACCACCCAACTTAG -3' (SEQ No.2)

The forward primer sequence was designed based on a specific base sequence in the fifth exon (corresponding to 1 to 25 of SEQ No.4), which is at the upstream of the deleted portion, while the reverse primer sequence was designed based on a specific base sequence in the fifth intron (corresponding to 408 to 432 of SEQ No.4), which is at the downstream of the deleted portion. The deleted portion is ranging from 173 to 256 of SEQ No.4.

The PCR was carried out by the following condition; firstly (1) 94°C for 2 minutes, next (2) 30 cycles of 94°C for 30 seconds / 65°C for 30 seconds / 72°C for 1 minute, followed by (3) 72°C for 7 minutes.

Different amplified fragments (PCR products) with different length are obtained by the above PCR method, according to the presence or absence of the deleted portion. The genotype of a test cattle (specimen) can be easily judged whether it is a L type or a S type, for example by electrophoresis of its PCR product with a marker.

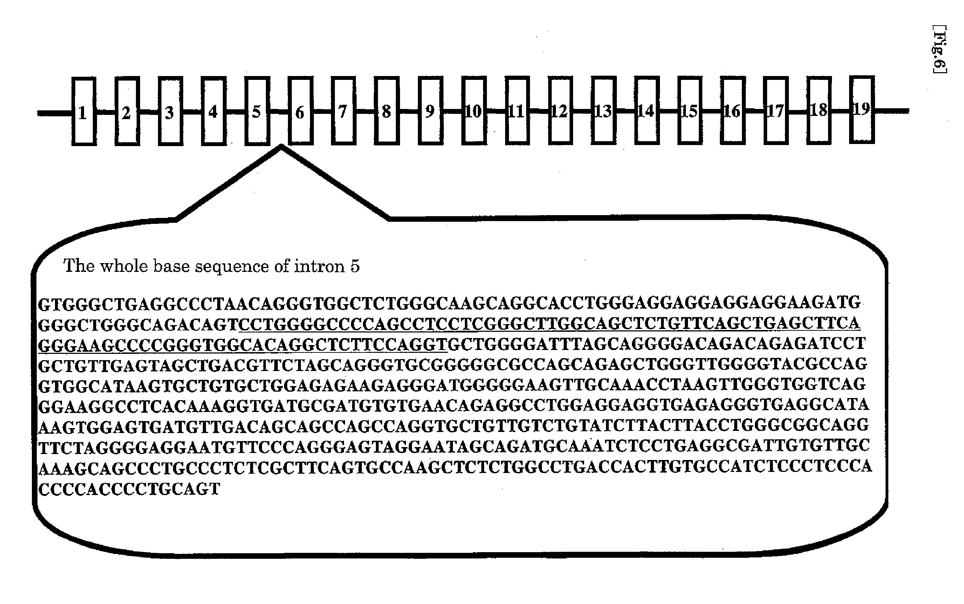

FIGS.7 and 8 show a result of the electrophoresis. As shown in these Figures, when only longer DNA band of 432 bp appeared, then the genotype of a test cattle is judged the type (LL) having homozygous L type. When only shorter DNA band of 348 bp appeared, then the genotype is judged the type (SS) having homozygous S type. When both of shorter and longer DNA bands appeared, then the genotype is judged the type (LS) having heterozygous L and S type. Thus, this PCR method enables simple and precise judgment of the genotype of bovine SREBP-1 gene.

### (3) Other embodiments of the present invention

The method of the present invention is not limited to the PCR method described in the above (2). For example, each condition for reaction, used reagents and primers, etc. can be variously changed, even when using the PCR method.

Of course, in addition to the PCR method, other methods may be used for the judgment (method) of the present invention. One example is a method by use of gene polymorphism detector tool (device) such as a DNA chip. This method is characterized by detection of hybridization signal between a probe and a genetic sample prepared from a test animal, using the DNA chip (or, similar device) that comprises, on a substrate, the probe to detect the presence or absence of the deleted portion. The probe can be designed based on a base sequence in the deleted portion, or its complementary sequence. Here, the word "DNA chip" principally means a synthesized-type DNA chip, comprising a synthesized oligonucleotide as the probe, but also includes an attached-type DNA microarray, comprising attached cDNA such as PCR product as the probe. The gene polymorphism detector tool, such as the DNA chip, is available to the method of the present invention, and also included in the present invention.

Other methods of the present invention include a method for detecting the presence or absence of the deleted portion, for example by PCR-RFLP (Restriction Fragment Length Polymorphism) method, and an amplification method other than the PCR, such as RCA method. In the PCR-RFLP method, a restriction enzyme may be used to cut a base sequence within the deleted portion. Genotyping is also possible by direct sequencing of amplified fragment using a sequencer, after DNA amplification.

The method of preparing a genetic sample from a test cattle is not limited to a specific method. It is a preferred approach to prepare a genetic sample from a hair of a test cattle because the cattle has no damages.

In addition to the above polymorphism in the fifth intron, other polymorphism (mutation) may have happened on the base sequence of bovine SREBP-1 gene, among animals belonging to Genus Bos (even among animals belonging to the Europeane cattle (Bos taurus)). Such polymorphism (mutation) on SREBP-1 gene may be associated with the unsaturated fatty acid percentage in beef fat, because the SREBP-1 gene expression level was correlated with the SCD gene expression level (FIG.5). The prediction of quality of beef, as well as the prediction of the unsaturated fatty acid content, may be available, by investigating such polymorphism (mutation). For example, when such polymorphism (mutation) is on the ORF (open reading frame) of SREBP-1 gene, then cDNA may be prepared from mRNA, in order to detect its polymorphism (mutation). When such polymorphism (mutation) is on the regulatory region at the upstream of the gene, then it is possible to know an effective genotype by comparing genomic sequences of that region.

### (4) Industrial applicability (utility) of the present invention

As mentioned above, the present invention is useful for various fields such as identification of cattle, selection of stud bull, livestock industry (feeding, breeding, reproduction, etc.), beef production, processing and distribution. For example, the present invention can be used for evaluation of beef quality from the beef cattle such as Japanese Black cattle. Moreover, the present invention can be used, through the above evaluation, for breeding and reproduction. That is, the present invention may be applied to the breeding strategy to produce a superiol herd which has the characteristics of high quality beef producer.

The unsaturated fatty acid content in beef fat is also associated with the cholesterol accumulation caused by beef intake. The present invention enables prediction of the unsaturated fatty acid content and therefore, it is also useful from the view point of human health. For example, development of the beef with lower melting point is important for production and development of a healthy beef (good beef for health), especially in the countries where beef is eaten very often, such as the Europe and the United States of America.

The present invention is available to various fields such as animal science and livestock industry, through selection and reproduction of a target cattle or reproduction by using sperm or fertilized egg, etc. For example, the present invention is available to reproduction using recombinant DNA technology etc., as well as various gene experiments. Also, the present invention is applicable to prenatal diagnosis (judgment) for reproduction. Such prenatal diagnosis includes the method to pick up embryo cell from the amniotic fluid of the uterus, to prepare genetic samples and to prepare selected eggs which will produce high quality beef.

More excellent judgment is available by combining the judgment method of the present invention with the judgment method based on the SCD genotype (for example, see Table 3 described later). In this method, if a cattle has both "S type" of the SREBP-1 genotype and "A type" of the SCD genotype (see JP 2004-261014), then its cattle can be evaluated to have good quality of beef, since the unsaturated fatty acid percentage in beef fat is higher and the melting point is lower. Especially, if a cattle has three or more alleles, as a total number of a number of S types and a number of A types, then its melting point in beef fat tends to be remarkably lower, and its unsaturated fatty acid percentage tends to be higher.

### (5) Bovine SREBP-1 gene and protein

Bovine SREBP-1 gene and protein are a new gene and protein, which were cloned and identified for the first time by the inventors and therefore, these also fall within the scope of the present invention.

The SREBP-1 protein of the present invention includes (a) a protein having an amino acid sequence of SEQ NO.6, and (b) a protein having an amino acid sequence obtained by deletion, replacement, and/or addition of one or several amino acids in the amino acid sequence of SEQ NO.6 and having a transcriptional regulatory activity by binding a SRE sequence.

"Bovine SREBP-1 protein" of the present invention may have additive polypeptides. An example of such additive polypeptides includes a case of having an epitope such as His, Myc, or flag.

The "deletion, replacement, and/or addition of one or several amino acids" means the deletion, replacement, and/or addition of a number of amino acids that can be deleted, replaced, and/or added by a conventional method such as site-specific mutagenesis. That is, the protein of (b) is a mutant protein of the protein of (a), and here "mutant" is used primarily to mean mutation artificially introduced by a conventional method, but it also includes similar naturally-occurring mutant proteins isolated and purified.

The SREBP-1 gene of the present invention, encoding the SREBP-1 protein of the present invention, includes (a) a DNA having a base sequence ranging from the 22nd base to the 3324th base, among a base sequence of SEQ NO.5, and (b) a DNA that has a base sequence hybridized under stringent conditions to a DNA having a base sequence that is complementary to at least 100 base sequence (preferably over 200 base sequence, more preferably over 500 base sequence) of SEQ NO.5, and that encodes a protein having a transcriptional regulatory activity by binding a SRE sequence.

"Gene" of the present invention is preferably cDNA, but it can also be RNA or genomic DNA. Furthermore, "gene" of the present invention may include a sequence other than coding region of bovine SREBP-1 protein, such as a sequence for untranslated region (UTR) or vector sequence (including expression vector sequence).

Hybridization under stringent conditions can be carried out using for example the method set forth in "Molecular Cloning: Cold Spring Harbor Laboratory Press, Current Protocols in Molecular Biology"; Wiley Interscience, and more specifically the following method is exemplified.

DNA molecules from a cDNA library or the like are transferred to a membrane and hybridized with labeled probes within a hybridization buffer. The composition of the hybridization buffer is for example 0.1 wt% SDS, 5 wt% dextran sulfate, 1/20 content blocking reagent, and 2 to 7 x SSC. One example for the blocking reagent is to use a solution of 100 x Denhardt's solution, 2% (weight/volume) Bovine serum albumin, 2% (weight/volume) polyvinyl pyrrolidone prepared at five times concentration and then diluted to 1/20 concentration.

The hybridization temperature is in the range of 40 to 80°C, more preferably 50 to 70°C, and most preferably 55 to 65°C, and after incubation of between several hours and overnight, the membrane is washed with a washing buffer. The washing temperature is preferably room temperature or the temperature during hybridization. The washing buffer composition is preferably a 0.1 to 6 x SSC + 0.1 wt% SDS solution, and the membrane is washed several times with the washing buffer while altering the SSC concentration. The DNA hybridized with the probe is then detected using the label attached to the probe.

The gene and protein of the present invention can be used widely for the research of the cattle, and can be also used for the production of a transgenic cattle. Such transgenic cattle can be produced with a known transgenic technology (for example, Science 1998 Oct23; 282(5389): 751-4), which comprises a step of introducing bovine SREBP-1 of the present invention into a cellular nucleus, a step of making this nucleus fuse together with a bovine egg, and a step of transplanting thus obtained embryo into a surrogate mother.

### Example

The present invention is described in detail below through Example thereof, but in no way is the present invention limited to this Example.

### [Example 1: Relation between the content of unsaturated fatty acid and the SCD gene expression level]

The content of unsaturated fatty acid is different among the cattle kinds, and as for Japanese Black Cattle, the content of unsaturated fatty acid is higher than that of the Holstein-Friesian, and the melting point in beef fat of Japanese Black Cattle is lower. We investigated a relation between the content of unsaturated fatty acid and the expression level of bovine SCD gene, in order to examine a possibility that the SCD gene expression level may be associated with the difference of the unsaturated fatty acid content among the cattle kinds.

After bovine mRNA was extracted and purified from cattle's adipose tissue or muscular tissue according to a conventional method, cDNA was synthesized with a reverse transcriptase. Using this cDNA as a template, real-time PCR was carried out with TaqMan™ probe, in order to measure the SCD gene expression level (the amount of mRNA). The used sequences of a forward primer, a reverse primer and a TaqMan™ probe are respectively shown in the following (1), (2) and (3).
(1) 5'- GTGATGTTCCAGAGGAGGTACTACAA -3' (SEQ No.7)
(2) 5'- AACGTTTCATCCCACAAGCATAGGA -3' (SEQ No.8)
(3) 5'- CCTGGTGTCCTGTTGTTGTGCTTCATCC -3' (SEQ No.9)

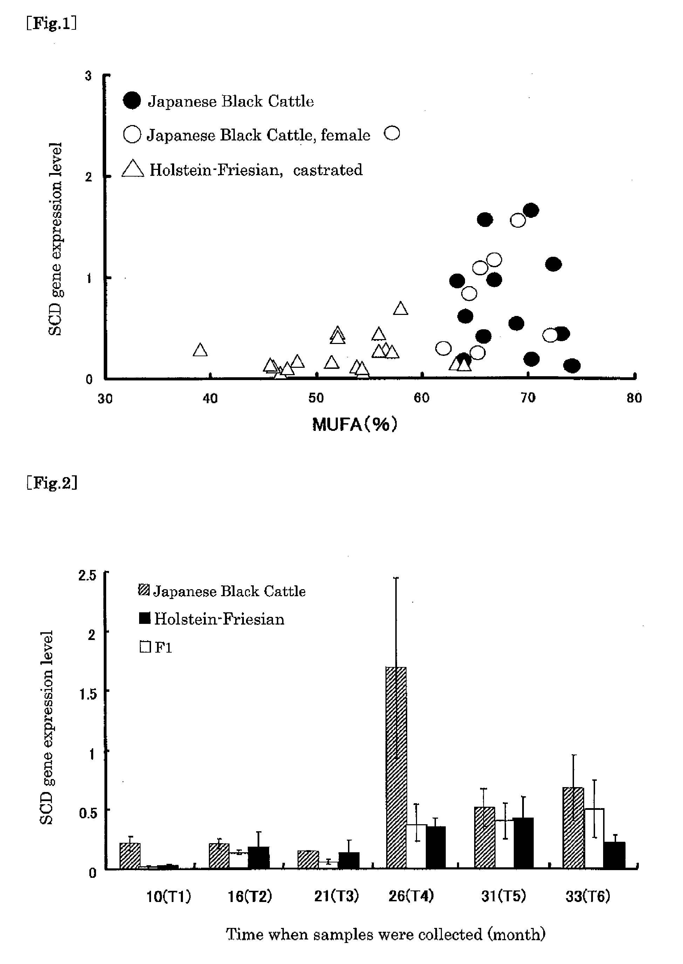

The result of the above experiment is shown in the graph of FIG.1, showing the relation between the SCD gene expression level (relative value) and the content (%) of mono-unsaturated fatty acid (MUFA) in the intermuscular adipose tissue. Also, Table 1, listed below, shows the investigation result of the amount of SCD mRNA and the MUFA content, in the muscle collected from the neck at the time of slaughter. The amount of SCD mRNA and the MUFA content were different between the cattle kinds. Little letters "a" and "b" in Table 1 (and Table 2 described later) show the significant difference by the risk 5% or less.

**Table 1**

| Kind | n | SCD mRNA | MUFA (%) |
|---|---|---|---|
| Japanese Black Cattle,castrated | 12 | 0.72±0.15^{a} | 64.5±1.0^{a} |
| Japanese Black Cattle,female | 8 | 1.00±0.26^{a} | 61.4±1.4^{a} |
| Holstein-Friesian,castrated | 19 | 0.23±0.04^{b} | 50.0±1.3^{b} |

| | | | |
|---|---|---|---|
| ^{ab} <0.05 | | | |

The result of the above experiment suggests that the SCD gene expression level is associated with the difference of the unsaturated fatty acid content among the cattle kinds. Furthermore, we carried out the following experiment in which different cattle kinds of the same month age were compared.

We investigated the MUFA content and the SCD gene expression level (the amount of mRNA) in both the muscle and the subcutaneous adipose tissue by using the samples, which were collected from the fattening cattle of 26 months of each kind of "Japanese Black Cattle", "Holstein-Friesian" and "F1(hybrid)" by the biopsy method. Table 2, listed below, shows the result.

**Table 2**

| Kind | n | SCD mRNA | | MUFA (%) |
|---|---|---|---|---|
| | | muscle | adipose tissue | |
| Japanese Black Cattle | 3 | 3.3±1.0^{a} | 132.1 ±34.1^{a} | 57.3±0.6^{a} |
| F1 | 3 | 1.1±0.3^{b} | 73.5 ±22.7^{ab} | 54.0±0.4^{ab} |
| Holstein-Friesian | 3 | 0.8±0.2^{b} | 39.5±12.9^{b} | 53.4±1.6^{b} |

| | | | | |
|---|---|---|---|---|
| ^{ab} P<0.05 | | | | |

In each cattle kind, the SCD gene expression level was higher in the adipose tissue than in the muscle, and it was associated with the MUFA content.

FIG.2 shows the result of examining a time change of the SCD gene expression level of each cattle kind. As shown in FIG.2, the SCD gene expression level of Japanese Black Cattle was especially high, and significantly different, at the month ages of 10 months and 26 months when the samples were collected.

FIG.3 shows the result of examining the relation between the MUFA content and the SCD gene expression level, about each kind of Japanese Black Cattle (JB), the Holstein-Friesian (Hols) and the F1 hybrid (F1). The samples of each kind were collected at the month ages of 10, 16, 21, 26 and 31 months, which are respectively shown as T1-T5 in FIG.3.

The results of the above-mentioned experiments suggest that the SCD gene expression level is associated with the difference of the unsaturated fatty acid content.

### [Example 2: Relation between the SCD gene expression level and the SREBP-1 gene expression level]

We paid attention to the SREBP-1 gene, as a factor affecting the SCD gene expression level, because of the following reasons; (1) as shown in FIG.4, there is a SRE sequence at the upstream of SCD gene, and the SREBP-1 is considered to bind to this SRE sequence and have an activity of regulating transcription of SCD gene; (2) there is a QTL (Quantitative Trait Loci), that is related to body fat, in the chromosomes 19 and 26, and SCD gene is located in the chromosome 26 while SREBP-1 gene is located in the chromosome 19. This suggests that SREBP-1 may be mainly involved in the control of the SCD gene expression.

In order to examine the relation between the SCD gene expression level and the SREBP-1 gene expression level (the amount of mRNA), real-time PCR was carried out similarly to the above-mentioned real-time PCR. The sequences of a forward primer, a reverse primer and a TaqMan™ probe, used to measure SREBP-1 mRNA, are respectively shown in the following (4), (5) and (6).
(4) 5'- TCAACTGTTCCGCGAACATC -3' (SEQ No.10)
(5) 5'- CATCTGAGAACTCCTTGTCCCCC -3'(SEQ No.11)
(6) 5'- TCTTGGAGCGAGCACTGAATTGCGT -3' (SEQ No. 12)

As a result of the above experiment, the amount of SCD mRNA was associated with the amount of SREBP-1 mRNA, as shown in FIG.5, and this result suggests that the SREBP-1 gene expression level may affect the SCD gene expression level.

### [Example 3: Relation between the MUFA content and the polymorphism found on SREBP-1 gene]

We investigated the presence of any mutation on SREBP-1 gene. As a result, we found out a polymorphism in the fifth intron (intron 5) between one genotype (L type) having the underlined 84 bases of FIG.6 while another genotype (S type) losing these 84 bases.

FIG.7 shows the result of judging the above SREBP-1 genotype, about each cattle kind of Japanese Black Cattle and the Holstein-Friesian. The genotype was judged by the above-mentioned PCR. Used reagents and condition for reaction, etc. are described above, and here omitted explanation thereof. As shown in FIG.7, all the Holstein-Friesians had only the L type in this investigation.

FIG.8 shows not only the result of determining the genotype of each test cattle by the above judgment method, but also the table summarizing the result of measuring the unsaturated fatty acid content and the melting point of each genotype. As shown in this table, the unsaturated fatty acid percentage in beef fat was the highest in the genotype (SS) and it was higher in the genotype (LS) than the genotype (LL), which is a result of analysis of variance in one factor of the SREBP-1 genotype. This table also shows the comparison of melting point. This result supports that, when the unsaturated fatty acid percentage is higher, the beef melting point is lower and the beef may have better quality, which gives better taste and texture.

### [Example 4: Judgment by the combination of SREBP-1 genotype and SCD genotype]

About each test cattle, SREBP-1 genotype was decided according to the above judgment method, while SCD genotype was decided according to the method described in JP2004-261014. In addition, we investigated the relation among the combination of these both genotypes, the unsaturated fatty acid content, and the melting point. The result is shown in Table 3.

**Table 3**

| genotype | n | MUFA(%) | melting point(°C) |
|---|---|---|---|
| LL/AA | 24 | 58.5±0.48 | 26.0±0.66 |
| LL/AV | 67 | 58.0±0.29 | 26.4±0.39 |
| LL/VV | 7 | 56.3±0.89 | 28.4±1.22 |
| LS/AA | 99 | 59.3±0.89 | 24.9±0.32 |
| LS/AV | 309 | 58.2±0.13 | 26.3±0.18 |
| LS/VV | 29 | 57.3±0.44 | 27.3±0.60 |
| SS/AA | 20 | 59.2±0.53 | 24.3±0.72 |
| SS/AV | 42 | 59.6±0.36 | 24.7±0.50 |
| SS/VV | 9 | 57.2±0.78 | 27.3±1.10 |

Based on this result, it can be evaluated that, if a cattle has a combination of "S type" as SREBP-1 genotype and "A type" as SCD genotype, then its cattle will produce better quality of beef. Especially, if a cattle has three or more alleles, as a total number of a number of "S type" alleles of SREBP-1 genotype and a number of "A-type" alleles of SCD genotype, then its cattle can be evaluated to produce better quality of beef, with higher unsaturated fatty acid content and lower melting point.

### Industrial Applicability

As mentioned above, the present invention is a method for evaluating quality of beef such as taste and texture, on the basis of the genotype of sterol regulatory element binding protein (SREBP-1), and useful for various fields such as identification of cattle, selection of stud bull, livestock industry (feeding, breeding, reproduction, etc.), beef production, processing and distribution.

## Claims

1. A method for evaluating the amount of the unsaturated fatty acid content in beef fat, on the basis of a genotype of sterol regulatory element binding protein (SREBP-1), to evaluate whether or not it is a cattle, from which better quality of beef with better taste and texture can be produced.

2. The method according to claim 1, comprising a step (a) of determining the genotype of SREBP-1 by examination of a polymorphism in a fifth intron of this gene, and a step (b) of evaluating a cattle with a "S type" allele to produce better quality of beef (better taste and texture), compared with a cattle with a "L type" allele, said "S type" allele has a shorter fifth intron while said "L type" allele has a longer fifth intron.

3. The method according to claim 2, comprising a step of amplifying a gene region including a deleted portion of 84 bases, related to the polymorphism in the fifth intron, by using a genomic DNA prepared from a test cattle as a template, and a step of determining the genotype of SREBP-1 by a length of an amplified fragment.

4. The method according to claim 3, adopting a PCR (polymerase chain reaction) by use of a forward primer having a base sequence shown in SEQ NO. 1 and a reverse primer having a base sequence shown in SEQ NO.2 in the amplification step.

5. The method according to claim 3, using a genomic DNA prepared from a hair of a test cattle as a template.

6. The method according to claim 2, examining the polymorphism in the fifth intron by use of a gene polymorphism detector device such as a DNA chip.

7. A method for evaluating the amount of the unsaturated fatty acid content in beef fat, on the basis of a combination of a genotype of sterol regulatory element binding protein (SREBP-1) and a genotype of stearoyl-CoA desaturase (SCD), to evaluate whether or not it is a cattle, from which better quality of beef with better taste and texture can be produced.

8. The method according to claim 7, comprising a step of evaluating a cattle to produce better quality of beef (better taste and texture), if the cattle has three or more alleles, as a total number of a number of "S type" alleles among the SREBP-1 genotype and a number of "A-type" alleles among the SCD genotype.

9. A bovine-derived SREBP-1 protein indicated by (a) or (b) below:
(a) a protein having an amino acid sequence of SEQ NO.6;
(b) a protein having an amino acid sequence obtained by deletion, replacement, or addition of one or several amino acids in the amino acid sequence of SEQ NO.6 and having a transcriptional regulatory activity by binding a SRE sequence.

10. A bovine-derived SREBP-1 gene, encoding any one protein set forth in claim 9.

11. The gene according to claim 10, said gene being a DNA indicated by (a) or (b) below:
(a) a DNA having a base sequence ranging from the 22nd base to the 3324th base, among a base sequence of SEQ NO.5;
(b) a DNA that has a base sequence hybridized under stringent conditions to a DNA having a base sequence that is complementary to at least 100 base sequence of SEQ NO.5, and that encodes a protein having a transcriptional regulatory activity by binding a SRE sequence.

12. A transgenic cattle, produced by introducing the gene according to claim 11.


SEQUENCE LISTING

<110>  THE NEW INDUSTRY RESEARCH ORGANIZATION

<120>  Method for evaluating beef taste and flavor by bovine SREBP-1 genotype

<130>  0162-WO01

<150>  JP 2005-028384
<151>  2005-02-04

<160>  12

<170>  PatentIn version 3.1

<210>  1
<211>  25
<212>  DNA
<213>  Bos taurus

<400>  1
ccacaacgcc atcgagaaac gctac                                              25

<210>  2
<211>  25
<212>  DNA
<213>  Bos taurus

<400>  2
ggccttccct gaccacccaa cttag                                              25

<210>  3
<211>  84
<212>  DNA
<213>  Bos taurus

<400>  3
cctggggccc cagcctcctc gggcttggca gctctgttca gctgagcttc agggaagccc      60

cgggtggcac aggctcttcc aggt                                               84

<210>  4
<211>  778
<212>  DNA
<213>  Bos taurus

<400>  4
ccacaacgcc atcgagaaac gctaccgctc ttccatcaat gacaagatcg ttgagctcaa      60

ggacctggtg gtgggcaccg aggccaaggt gtgggctgag gccctaacag ggtggctctg     120

ggcaagcagg cacctgggag gaggaggagg aagatggggc tgggcagaca gtcctggggc     180

cccagcctcc tcgggcttgg cagctctgtt cagctgagct tcagggaagc cccgggtggc     240

acaggctctt ccaggtgctg gggatttagc aggggacaga cagagatcct gctgttgagt     300

agctgacgtt ctagcagggt gcgggggcgc cagcagagct gggttggggt acgccaggtg     360

gcataagtgc tgtgctggag agaagaggga tgggggaagt tgcaaaccta agttgggtgg     420

tcagggaagg cctcacaaag gtgatgcgat gtgtgaacag aggcctggag gaggtgagag     480

ggtgaggcat aaagtggagt gatgttgaca gcagccagcc aggtgctgtt gtctgtatct     540

tacttacctg ggcggcaggt tctaggggag gaatgttccc agggagtagg aatagcagat 600

gcaaatctcc tgaggcgatt gtgttgcaaa gcagccctgc cctctcgctt cagtgccaag 660

ctctctggcc tgaccacttg tgccatctcc ctcccacccc accccctgcag ttgaataaat 720

ctgccgtctt gcgcaaggcc atcgactaca tccgcttcct tcagcacagc aaccagaa 778

<210> 5
<211> 3574
<212> DNA
<213> Bos taurus

<220>
<221> CDS
<222> (22)..(3324)
<223>

<400> 5
ccgagaggag gtgactgcgc c atg gac gag cca ccc ttc aac gaa gcg gcc 51
Met Asp Glu Pro Pro Phe Asn Glu Ala Ala
1               5                   10

ttg gag ctg gcg ctg gcc gag ccg tgc gag ctg gac gcg gcg ctg ctg 99
Leu Glu Leu Ala Leu Ala Glu Pro Cys Glu Leu Asp Ala Ala Leu Leu
                15                  20                  25

acc gac ata gca gac atg ctt cag ctc atc aac aac caa gac agc gaa 147
Thr Asp Ile Ala Asp Met Leu Gln Leu Ile Asn Asn Gln Asp Ser Glu
            30                  35                  40

ttc ccg ggc cta ttc gac ccg ccc tac gct ggg ggc gga gca ggg acc 195
Phe Pro Gly Leu Phe Asp Pro Pro Tyr Ala Gly Gly Gly Ala Gly Thr
        45                  50                  55

aca gac cct gcc agt cct gac gcc agc tcc ccg ggc agc ctg tcc cca 243
Thr Asp Pro Ala Ser Pro Asp Ala Ser Ser Pro Gly Ser Leu Ser Pro
    60                  65                  70

cct cct tcc acg atg agc tcc tca ctt gaa ggc ttc ctg ggg gtg acc 291
Pro Pro Ser Thr Met Ser Ser Ser Leu Glu Gly Phe Leu Gly Val Thr
75                  80                  85                  90

aag gcg aca ccc cca ccc ttg tcc cct ccc cag cct gcg ccc acc ccc 339
Lys Ala Thr Pro Pro Pro Leu Ser Pro Pro Gln Pro Ala Pro Thr Pro
                95                  100                 105

ctg aag ata tac cca tct gtg cct gcc ttc tcc ccg gtg cct ggg atc 387
Leu Lys Ile Tyr Pro Ser Val Pro Ala Phe Ser Pro Val Pro Gly Ile
            110                 115                 120

aag gag gag cca gcg ccc ctg act gtc ctc cag ccc acc acc ccc cag 435
Lys Glu Glu Pro Ala Pro Leu Thr Val Leu Gln Pro Thr Thr Pro Gln
        125                 130                 135

ccc ctg cct gga gct ctc ctg ccg cag agt gtt gcg gcc acc acc cca 483
Pro Leu Pro Gly Ala Leu Leu Pro Gln Ser Val Ala Ala Thr Thr Pro
    140                 145                 150

ccg cag ttc agc tct gct ccc att gtg ggc tac ccc agc cct ccg gga 531
Pro Gln Phe Ser Ser Ala Pro Ile Val Gly Tyr Pro Ser Pro Pro Gly
155                 160                 165                 170

ggc ttc aca gca ggg acc cct ccg ggg agc agc tcg cag tca ctg gct 579
Gly Phe Thr Ala Gly Thr Pro Pro Gly Ser Ser Ser Gln Ser Leu Ala
                175                 180                 185

ggc ctg cca ctg gct tcc ctg cca ggg gtc ccg ccc gtc tcc ttg cac 627
Gly Leu Pro Leu Ala Ser Leu Pro Gly Val Pro Pro Val Ser Leu His
            190                 195                 200

agc cag gtt cag agc gcg gcc ccc cag cag ctg ttg aca gcc aca gcc 675
Ser Gln Val Gln Ser Ala Ala Pro Gln Gln Leu Leu Thr Ala Thr Ala
        205                 210                 215

acc ccc acg gtg gcc cct gga gca acc gct gtg acc tcc cag ata cag 723
Thr Pro Thr Val Ala Pro Gly Ala Thr Ala Val Thr Ser Gln Ile Gln
    220                 225                 230

cag gtc ccg gtc ctg ctg cag ccc cac ttc atc aag gca gac tcg ttg 771
Gln Val Pro Val Leu Leu Gln Pro His Phe Ile Lys Ala Asp Ser Leu
235                 240                 245                 250

ctc ctg acg acc gtg aaa aca gat gtg gga gcc ccc ttg aaa gcg gca 819
Leu Leu Thr Thr Val Lys Thr Asp Val Gly Ala Pro Leu Lys Ala Ala
                255                 260                 265

ggc atc cgc tcc ctg ggc cct ggc act gct gtt cag gca gcg ccc ttg 867
Gly Ile Arg Ser Leu Gly Pro Gly Thr Ala Val Gln Ala Ala Pro Leu
            270                 275                 280

cag acc ctg gtg agt ggc ggg gcc atc ctg gcc acg gtg cca ctg gta 915
Gln Thr Leu Val Ser Gly Gly Ala Ile Leu Ala Thr Val Pro Leu Val
        285                 290                 295

gta gac act gac aag ctg ccc atc aac cga ctt gcc agt ggc aag gct 963
Val Asp Thr Asp Lys Leu Pro Ile Asn Arg Leu Ala Ser Gly Lys Ala
    300                 305                 310

cca ggc tcg gcg cag agc cgc ggc gag aag cgt aca gcc cac aac gcc 1011
Pro Gly Ser Ala Gln Ser Arg Gly Glu Lys Arg Thr Ala His Asn Ala
315                 320                 325                 330

atc gag aaa cgc tac cgc tct tcc atc aat gac aag atc gtt gag ctc 1059
Ile Glu Lys Arg Tyr Arg Ser Ser Ile Asn Asp Lys Ile Val Glu Leu
                335                 340                 345

aag gac ctg gtg gtg ggc acc gag gcc aag ttg aat aaa tct gcc gtc 1107
Lys Asp Leu Val Val Gly Thr Glu Ala Lys Leu Asn Lys Ser Ala Val
            350                 355                 360

ttg cgc aag gcc atc gac tac atc cgc ttc ctt cag cac agc aac cag 1155
Leu Arg Lys Ala Ile Asp Tyr Ile Arg Phe Leu Gln His Ser Asn Gln
        365                 370                 375

aag ctc aag cag gag aac ctg agt ctg cgc act gct gtc cac aaa agc 1203
Lys Leu Lys Gln Glu Asn Leu Ser Leu Arg Thr Ala Val His Lys Ser
    380                 385                 390

aaa tcg ctg aag gac ctg gtg tcg gct tgt ggc agt gga ggg agc aca 1251
Lys Ser Leu Lys Asp Leu Val Ser Ala Cys Gly Ser Gly Gly Ser Thr
395                 400                 405                 410

gat gtg ccc atg gag ggc atg aag cca gag gtg gtc gac acc ctg agt 1299
Asp Val Pro Met Glu Gly Met Lys Pro Glu Val Val Asp Thr Leu Ser
                415                 420                 425

cca ccc ccc tca gat gcc ggc tcg ccc tcc cag agc agc ccc ttg tcc 1347
Pro Pro Pro Ser Asp Ala Gly Ser Pro Ser Gln Ser Ser Pro Leu Ser
            430                 435                 440

ctt ggc agc agg ggc agt agc agc ggt gga agt ggc agt gac tcg gag 1395
Leu Gly Ser Arg Gly Ser Ser Ser Gly Gly Ser Gly Ser Asp Ser Glu
        445                 450                 455

ccc gac agc ccg gtc ttt gag gac ggc cag gtg aat cca gag ccg ctg 1443
Pro Asp Ser Pro Val Phe Glu Asp Gly Gln Val Asn Pro Glu Pro Leu
    460                 465                 470

cct gct ccc cac agc cag ggc atg ctg gac cgc tct cgc ctg gcc ctg 1491
Pro Ala Pro His Ser Gln Gly Met Leu Asp Arg Ser Arg Leu Ala Leu
475                 480                 485                 490

tgc gca ctc gtc ttc ctc tgt ctc tcc tgc aac ccc ttg gcc tcc ctg 1539
Cys Ala Leu Val Phe Leu Cys Leu Ser Cys Asn Pro Leu Ala Ser Leu
                495                 500                 505

ctg ggt agc cgg ggt cct gct ggc ccc tcc gac acc acc agc atc aac 1587
Leu Gly Ser Arg Gly Pro Ala Gly Pro Ser Asp Thr Thr Ser Ile Asn
            510                 515                 520

cac ggt ccc agg cgc agc atg ctg ggt gct gag ggc aga gat ggt cct 1635
His Gly Pro Arg Arg Ser Met Leu Gly Ala Glu Gly Arg Asp Gly Pro
        525                 530                 535

ggc tgg gcc cca tgg ctg ctg ccc cca ctg gtc tgg ctg atg aat ggg 1683
Gly Trp Ala Pro Trp Leu Leu Pro Pro Leu Val Trp Leu Met Asn Gly
    540                 545                 550

ctg ctg gtg ctc ttc tcc ttg gca ctt ctc ttt gtc tat gga gaa cca 1731
Leu Leu Val Leu Phe Ser Leu Ala Leu Leu Phe Val Tyr Gly Glu Pro
555                 560                 565                 570

gtc act cgg ccc cac tca tgc ccc gcc gtg cac ttc tgg agg cat cgc 1779
Val Thr Arg Pro His Ser Cys Pro Ala Val His Phe Trp Arg His Arg
                575                 580                 585

aag cag gct gac ctg gac ctg gcc agg ggg gac ttt gcc cag gct gcc 1827
Lys Gln Ala Asp Leu Asp Leu Ala Arg Gly Asp Phe Ala Gln Ala Ala
            590                 595                 600

cag cag ctg tgg ctg gcc ctg cgg gcc ttg ggc cgg cct ctg ccc acc 1875
Gln Gln Leu Trp Leu Ala Leu Arg Ala Leu Gly Arg Pro Leu Pro Thr
        605                 610                 615

tcc cac ctg gac ctg gcc tgc agc ctg ctc tgg agc ctc atc cgc cac 1923
Ser His Leu Asp Leu Ala Cys Ser Leu Leu Trp Ser Leu Ile Arg His
    620                 625                 630

ctg ctg caa cgt ctc tgg gtg ggc cgc tgg ctg gcc ggc tgg gca ggg 1971
Leu Leu Gln Arg Leu Trp Val Gly Arg Trp Leu Ala Gly Trp Ala Gly
635                 640                 645                 650

ggc cta cgg agg gac agg gcc cta cag gca gat gct cgc acc agt gcc 2019
Gly Leu Arg Arg Asp Arg Ala Leu Gln Ala Asp Ala Arg Thr Ser Ala
                655                 660                 665

cgt gac gca gcc ctc gtc tac cac aag ctg cac cag ctg cac acc atg 2067
Arg Asp Ala Ala Leu Val Tyr His Lys Leu His Gln Leu His Thr Met
            670                 675                 680

gga aag tac tca ggt ggg cac ctc gct gct gcc aac ctg gca ctg agt 2115
Gly Lys Tyr Ser Gly Gly His Leu Ala Ala Ala Asn Leu Ala Leu Ser
        685                 690                 695

gcc ctg aac ctg gcc gag tgc gct gga gat gct gtg tcc gtg gcc acg 2163
Ala Leu Asn Leu Ala Glu Cys Ala Gly Asp Ala Val Ser Val Ala Thr
    700                 705                 710

ctg gct gag atc tac gtg gcc gcc gca ctc agg gtc aag gcc agt ctg 2211
Leu Ala Glu Ile Tyr Val Ala Ala Ala Leu Arg Val Lys Ala Ser Leu
715                 720                 725                 730

ccc cgg gcc ttg cat ttt ctg aca cgc ttc ttc ctg agc agt gct cgc 2259
Pro Arg Ala Leu His Phe Leu Thr Arg Phe Phe Leu Ser Ser Ala Arg
                735                 740                 745

cag gcc tgc ctg gca cag agc ggc tca gtg ccc ctt gcc atg cag tgg 2307
Gln Ala Cys Leu Ala Gln Ser Gly Ser Val Pro Leu Ala Met Gln Trp
            750                 755                 760

ctc tgc cac cct gtg ggc cac cgt ttc ttc gtg gat ggc aac tgg gcc 2355
Leu Cys His Pro Val Gly His Arg Phe Phe Val Asp Gly Asn Trp Ala
        765                 770                 775

ctg tgc agc gcc ccg agg gac agc ttg tac agc gtg gct ggg aac cca 2403
Leu Cys Ser Ala Pro Arg Asp Ser Leu Tyr Ser Val Ala Gly Asn Pro
    780                 785                 790

gtg gat ccc ctg gcc cag gtg act caa ctg ttc cgc gaa cat ctc ttg 2451
Val Asp Pro Leu Ala Gln Val Thr Gln Leu Phe Arg Glu His Leu Leu
795                 800                 805                 810

gag cga gca ctg aat tgc gtg gcc cag ccc agc ccc agt cct gga tca 2499
Glu Arg Ala Leu Asn Cys Val Ala Gln Pro Ser Pro Ser Pro Gly Ser
                815                 820                 825

gcc gag ggg gac aag gag ttc tca gat gcc ctg ggg tac ctg cag ctg 2547
Ala Glu Gly Asp Lys Glu Phe Ser Asp Ala Leu Gly Tyr Leu Gln Leu
            830                 835                 840

ctg aac agc tgt tcg gac gtg gcc gga gct ctt acc tgc agc ttc tcc 2595
Leu Asn Ser Cys Ser Asp Val Ala Gly Ala Leu Thr Cys Ser Phe Ser
        845                 850                 855

atc agc tcc agc atg gct gcc acc ccc ggc aca gac ccg gtg gcc aag 2643
Ile Ser Ser Ser Met Ala Ala Thr Pro Gly Thr Asp Pro Val Ala Lys
    860                 865                 870

tgg tgg gcc tct ctg aca gct gtg gtg acc cac tgg ctt cgg cgg gat 2691
Trp Trp Ala Ser Leu Thr Ala Val Val Thr His Trp Leu Arg Arg Asp
875                 880                 885                 890

gag gag gca gcc gag agg ctg tac cca ttg gtg gag cac ctg ccc cgt 2739
Glu Glu Ala Ala Glu Arg Leu Tyr Pro Leu Val Glu His Leu Pro Arg
                895                 900                 905

gcc ctg cag gag tcc gag aaa ccc cta ccc agg gcg gct ctg cac tcc 2787
Ala Leu Gln Glu Ser Glu Lys Pro Leu Pro Arg Ala Ala Leu His Ser
            910                 915                 920

ttc aag gct gcc cgg acc att cta ggc cgc ggg aag gct gag tct ggc 2835
Phe Lys Ala Ala Arg Thr Ile Leu Gly Arg Gly Lys Ala Glu Ser Gly
        925                 930                 935

cca gcc agc ctg gca atg tgt gag aag gcc agt ggg tac ctg cag gac 2883
Pro Ala Ser Leu Ala Met Cys Glu Lys Ala Ser Gly Tyr Leu Gln Asp
    940                 945                 950

agc ctg gcc acc aca cca gct gac agc tcc att gac aag gcc ttg cag 2931
Ser Leu Ala Thr Thr Pro Ala Asp Ser Ser Ile Asp Lys Ala Leu Gln
955                 960                 965                 970

ctg ctc ctg tgt gac ctg ctc ctt gtg gcg cgc acc agc ctc tgg cag 2979
Leu Leu Leu Cys Asp Leu Leu Leu Val Ala Arg Thr Ser Leu Trp Gln
                975                 980                 985

cgg cag aag ctg ccg gca ccc acc cag gcc tcg cag ggc cct gga ggt 3027
Arg Gln Lys Leu Pro Ala Pro Thr Gln Ala Ser Gln Gly Pro Gly Gly
            990                 995                 1000

ggg gcc cag gcc tct gcc ctc gag ctt cgt ggt ttc cag agg gac 3072
Gly Ala Gln Ala Ser Ala Leu Glu Leu Arg Gly Phe Gln Arg Asp
        1005                1010                1015

ctg agt ggc ctg agg cgt ctg gca cag aat gtt cgg cct gcc atg 3117
Leu Ser Gly Leu Arg Arg Leu Ala Gln Asn Val Arg Pro Ala Met
        1020                1025                1030

cgg agg gta ttc cta cat gaa gcc act gcc cga ctg atg gca ggg 3162
Arg Arg Val Phe Leu His Glu Ala Thr Ala Arg Leu Met Ala Gly
        1035                1040                1045

gcc agc cca gcg cgg aca aaa cag ctt ctg gat cgc agc ctg agg 3207
Ala Ser Pro Ala Arg Thr Lys Gln Leu Leu Asp Arg Ser Leu Arg
        1050                1055                1060

agg agg gtc ggc tcc cgc act cgg cgc gcg gtg gcc agg ata gtg 3252
Arg Arg Val Gly Ser Arg Thr Arg Arg Ala Val Ala Arg Ile Val
        1065                1070                1075

ctg gcc cct ggt ggc cgg ctg ggg aat tac cgg ggc ctg ccg ccg 3297
Leu Ala Pro Gly Gly Arg Leu Gly Asn Tyr Arg Gly Leu Pro Pro
        1080                1085                1090

ccg ccg gat gtc aag tgc tcc cag tga caacgctcct gggtgtcatg 3344
Pro Pro Asp Val Lys Cys Ser Gln
        1095                1100

ggccccagtc cgcagctctc cccctccctc cagagagaag agaggggtgc atttcaccga 3404

accgagggac cctacccact gtgcccccct cccatctagg gagacctgtg catagtgtag 3464

atcggagtgc acagcctcct gggctcaagg gtcaggcgtt actttgcttt tgcagacttt 3524

atttttatag gttgagaggt ttgtaaaaaa aaaaaaaaaa aaaaaaaaaa 3574

<210> 6
<211> 1100
<212> PRT
<213> Bos taurus

<400> 6

Met Asp Glu Pro Pro Phe Asn Glu Ala Ala Leu Glu Leu Ala Leu Ala
1               5                   10                  15

Glu Pro Cys Glu Leu Asp Ala Ala Leu Leu Thr Asp Ile Ala Asp Met
            20                  25                  30

Leu Gln Leu Ile Asn Asn Gln Asp Ser Glu Phe Pro Gly Leu Phe Asp
        35                  40                  45

Pro Pro Tyr Ala Gly Gly Gly Ala Gly Thr Thr Asp Pro Ala Ser Pro
    50                  55                  60

Asp Ala Ser Ser Pro Gly Ser Leu Ser Pro Pro Pro Ser Thr Met Ser
65                  70                  75                  80

Ser Ser Leu Glu Gly Phe Leu Gly Val Thr Lys Ala Thr Pro Pro Pro
                85                  90                  95

Leu Ser Pro Pro Gln Pro Ala Pro Thr Pro Leu Lys Ile Tyr Pro Ser
            100                 105                 110

Val Pro Ala Phe Ser Pro Val Pro Gly Ile Lys Glu Glu Pro Ala Pro
        115                 120                 125

Leu Thr Val Leu Gln Pro Thr Thr Pro Gln Pro Leu Pro Gly Ala Leu
    130                 135                 140

Leu Pro Gln Ser Val Ala Ala Thr Thr Pro Pro Gln Phe Ser Ser Ala
145                 150                 155                 160

Pro Ile Val Gly Tyr Pro Ser Pro Pro Gly Gly Phe Thr Ala Gly Thr
                165                 170                 175

Pro Pro Gly Ser Ser Ser Gln Ser Leu Ala Gly Leu Pro Leu Ala Ser
            180                 185                 190

Leu Pro Gly Val Pro Pro Val Ser Leu His Ser Gln Val Gln Ser Ala
        195                 200                 205

Ala Pro Gln Gln Leu Leu Thr Ala Thr Ala Thr Pro Thr Val Ala Pro
    210                 215                 220

Gly Ala Thr Ala Val Thr Ser Gln Ile Gln Gln Val Pro Val Leu Leu
225                 230                 235                 240

Gln Pro His Phe Ile Lys Ala Asp Ser Leu Leu Leu Thr Thr Val Lys
                245                 250                 255

Thr Asp Val Gly Ala Pro Leu Lys Ala Ala Gly Ile Arg Ser Leu Gly
            260                 265                 270

Pro Gly Thr Ala Val Gln Ala Ala Pro Leu Gln Thr Leu Val Ser Gly
        275                 280                 285

Gly Ala Ile Leu Ala Thr Val Pro Leu Val Val Asp Thr Asp Lys Leu
    290                 295                 300

Pro Ile Asn Arg Leu Ala Ser Gly Lys Ala Pro Gly Ser Ala Gln Ser
305                 310                 315                 320

Arg Gly Glu Lys Arg Thr Ala His Asn Ala Ile Glu Lys Arg Tyr Arg
                325                 330                 335

Ser Ser Ile Asn Asp Lys Ile Val Glu Leu Lys Asp Leu Val Val Gly
            340                 345                 350

Thr Glu Ala Lys Leu Asn Lys Ser Ala Val Leu Arg Lys Ala Ile Asp
        355                 360                 365

Tyr Ile Arg Phe Leu Gln His Ser Asn Gln Lys Leu Lys Gln Glu Asn
    370                 375                 380

Leu Ser Leu Arg Thr Ala Val His Lys Ser Lys Ser Leu Lys Asp Leu
385                 390                 395                 400

Val Ser Ala Cys Gly Ser Gly Gly Ser Thr Asp Val Pro Met Glu Gly
                405                 410                 415

Met Lys Pro Glu Val Val Asp Thr Leu Ser Pro Pro Pro Ser Asp Ala
            420                 425                 430

Gly Ser Pro Ser Gln Ser Ser Pro Leu Ser Leu Gly Ser Arg Gly Ser
        435                 440                 445

Ser Ser Gly Gly Ser Gly Ser Asp Ser Glu Pro Asp Ser Pro Val Phe
    450                 455                 460

Glu Asp Gly Gln Val Asn Pro Glu Pro Leu Pro Ala Pro His Ser Gln
465                 470                 475                 480

Gly Met Leu Asp Arg Ser Arg Leu Ala Leu Cys Ala Leu Val Phe Leu
                485                 490                 495

Cys Leu Ser Cys Asn Pro Leu Ala Ser Leu Leu Gly Ser Arg Gly Pro
            500                 505                 510

Ala Gly Pro Ser Asp Thr Thr Ser Ile Asn His Gly Pro Arg Arg Ser
        515                 520                 525

Met Leu Gly Ala Glu Gly Arg Asp Gly Pro Gly Trp Ala Pro Trp Leu
    530                 535                 540

Leu Pro Pro Leu Val Trp Leu Met Asn Gly Leu Leu Val Leu Phe Ser
545                 550                 555                 560

Leu Ala Leu Leu Phe Val Tyr Gly Glu Pro Val Thr Arg Pro His Ser
                565                 570                 575

Cys Pro Ala Val His Phe Trp Arg His Arg Lys Gln Ala Asp Leu Asp
            580                 585                 590

Leu Ala Arg Gly Asp Phe Ala Gln Ala Ala Gln Gln Leu Trp Leu Ala
        595                 600                 605

Leu Arg Ala Leu Gly Arg Pro Leu Pro Thr Ser His Leu Asp Leu Ala
    610                 615                 620

Cys Ser Leu Leu Trp Ser Leu Ile Arg His Leu Leu Gln Arg Leu Trp
625                 630                 635                 640

Val Gly Arg Trp Leu Ala Gly Trp Ala Gly Gly Leu Arg Arg Asp Arg
                645                 650                 655

Ala Leu Gln Ala Asp Ala Arg Thr Ser Ala Arg Asp Ala Ala Leu Val
            660                 665                 670

Tyr His Lys Leu His Gln Leu His Thr Met Gly Lys Tyr Ser Gly Gly
        675                 680                 685

His Leu Ala Ala Ala Asn Leu Ala Leu Ser Ala Leu Asn Leu Ala Glu
    690                 695                 700

Cys Ala Gly Asp Ala Val Ser Val Ala Thr Leu Ala Glu Ile Tyr Val
705                 710                 715                 720

Ala Ala Ala Leu Arg Val Lys Ala Ser Leu Pro Arg Ala Leu His Phe
                725                 730                 735

Leu Thr Arg Phe Phe Leu Ser Ser Ala Arg Gln Ala Cys Leu Ala Gln
            740                 745                 750

Ser Gly Ser Val Pro Leu Ala Met Gln Trp Leu Cys His Pro Val Gly
        755                 760                 765

His Arg Phe Phe Val Asp Gly Asn Trp Ala Leu Cys Ser Ala Pro Arg
    770                 775                 780

Asp Ser Leu Tyr Ser Val Ala Gly Asn Pro Val Asp Pro Leu Ala Gln
785                 790                 795                 800

Val Thr Gln Leu Phe Arg Glu His Leu Leu Glu Arg Ala Leu Asn Cys
                805                 810                 815

Val Ala Gln Pro Ser Pro Ser Pro Gly Ser Ala Glu Gly Asp Lys Glu
            820                 825                 830

Phe Ser Asp Ala Leu Gly Tyr Leu Gln Leu Leu Asn Ser Cys Ser Asp
        835                 840                 845

Val Ala Gly Ala Leu Thr Cys Ser Phe Ser Ile Ser Ser Ser Met Ala
    850                 855                 860

Ala Thr Pro Gly Thr Asp Pro Val Ala Lys Trp Trp Ala Ser Leu Thr
865                 870                 875                 880

Ala Val Val Thr His Trp Leu Arg Arg Asp Glu Glu Ala Ala Glu Arg
                885                 890                 895

Leu Tyr Pro Leu Val Glu His Leu Pro Arg Ala Leu Gln Glu Ser Glu
            900                 905                 910

Lys Pro Leu Pro Arg Ala Ala Leu His Ser Phe Lys Ala Ala Arg Thr
        915                 920                 925

Ile Leu Gly Arg Gly Lys Ala Glu Ser Gly Pro Ala Ser Leu Ala Met
    930                 935                 940

Cys Glu Lys Ala Ser Gly Tyr Leu Gln Asp Ser Leu Ala Thr Thr Pro
945                 950                 955                 960

Ala Asp Ser Ser Ile Asp Lys Ala Leu Gln Leu Leu Leu Cys Asp Leu
                965                 970                 975

Leu Leu Val Ala Arg Thr Ser Leu Trp Gln Arg Gln Lys Leu Pro Ala
            980                 985                 990

Pro Thr Gln Ala Ser Gln Gly Pro Gly Gly Gly Ala Gln Ala Ser Ala
        995                 1000                1005

Leu Glu Leu Arg Gly Phe Gln Arg Asp Leu Ser Gly Leu Arg Arg
    1010                1015                1020

Leu Ala Gln Asn Val Arg Pro Ala Met Arg Arg Val Phe Leu His
    1025                1030                1035

Glu Ala Thr Ala Arg Leu Met Ala Gly Ala Ser Pro Ala Arg Thr
    1040                1045                1050

Lys Gln Leu Leu Asp Arg Ser Leu Arg Arg Arg Val Gly Ser Arg
    1055                1060                1065

Thr Arg Arg Ala Val Ala Arg Ile Val Leu Ala Pro Gly Gly Arg
    1070                1075                1080

Leu Gly Asn Tyr Arg Gly Leu Pro Pro Pro Pro Asp Val Lys Cys
    1085                1090                1095

Ser Gln
    1100

<210> 7
<211> 26
<212> DNA
<213> Bos taurus

<400> 7
gtgatgttcc agaggaggta ctacaa 26

<210> 8
<211> 25
<212> DNA
<213> Bos taurus

<400> 8
aacgtttcat cccacaagca tagga 25

<210> 9
<211> 28
<212> DNA
<213> Bos taurus

<400> 9
cctggtgtcc tgttgttgtg cttcatcc 28

<210> 10
<211> 20
<212> DNA
<213> Bos taurus

<400> 10
tcaactgttc cgcgaacatc 20

<210> 11
<211> 23
<212> DNA
<213> Bos taurus

<400> 11
catctgagaa ctccttgtcc ccc 23

<210> 12
<211> 25
<212> DNA
<213> Bos taurus

<400> 12
tcttggagcg agcactgaat tgcgt 25